# EUROPEAN PATENT APPLICATION

(11) **EP 4 021 150 A1**
(43) Date of publication of application: **29.06.2022**
(21) Application number: 21214969.4
(22) Date of filing: 16.12.2021
(51) Int. Cl.: H05H 1/24, C02F 1/78

(54) **OZONE GENERATOR**

(30) Priority: 22.12.2020 GB 202020395
(71) Applicant: Sun Hydraulics (China) Co., Ltd., Kunshan City, Jiangsu 215300 (CN)
(72) Inventor: CHEN, Zhihai, Guangming District, Shenzhen, Guangdong (CN); CARLIN, Robert, Chesterfield, Derbyshire, S41 9RT (GB)
(74) Representative: Murgitroyd & Company

(57) **Abstract**

There is provided an ozone generator for a hot tub, the ozone generator comprising an elongate body comprising an inlet and an outlet to define an flow path therealong and first and second electrodes spaced apart to define a gap therebetween, said gap being provided along the flow path. The ozone generator is configured to apply an electrical charge to the first and second electrodes, in use, to generate an electric arc discharge across the gap. The ozone generator further comprises a shielding arrangement configured to isolate the first and second electrodes from the electric arc discharge generated during use. By isolating the electrodes from the electric arc discharge, they are shielded from surface oxidation and subsequent corrosion caused by the electric arc discharge, thereby improving service life.

## Description

### FIELD

The present teachings relates to an ozone generator for a hot tub and/or hydrotherapy system, and to a hot tub and/or hydrotherapy system.

### BACKGROUND

Modern hot-tub and hydrotherapy systems typically feature an ozone generator to help reduce the presence of impurities and microbial life within the spa water of the system. When ozone is infused into water, it is able to effectively kill many kinds of bacteria and viruses without the need for adding excessive amounts of chemicals. Ozone can also cause dissolved solids within the spa water to aggregate or clump together so that they can be easily removed from the water via a filter.

One type of ozone generator commonly used in hot-tub and hydrotherapy systems is a "corona discharge unit" which applies a voltage to a pair of metal electrodes to generate an electric arc discharge between the electrodes. The electric arc discharge breaks down any oxygen molecules (O₂) present between the electrodes into pairs of oxygen atoms (O). These oxygen atoms (O) then react with further oxygen molecules (O₂) to form ozone (O₃) ready for introduction into the spa water of the hot-tub or hydrotherapy system. The ionizing nature of the electric arc discharge can lead to issues with oxidation and corrosion of parts of the ozone generator which adversely effects service life.

The present teachings seek to overcome or at least mitigate one or more problems associated with the prior art.

### SUMMARY

A first aspect of the teachings provides an ozone generator for a hot tub, the ozone generator comprising an elongate body comprising an inlet and an outlet to define a flow path therealong and first and second electrodes spaced apart to define a gap therebetween, said gap being provided along the flow path, wherein the ozone generator is configured to apply an electrical charge to the first and second electrodes, in use, to generate an electric arc discharge across the gap and wherein the ozone generator comprises a shielding arrangement configured to isolate the first and second electrodes from the electric arc discharge generated during use.

Advantageously, by isolating the electrodes from the electric arc discharge, they are shielded from surface oxidation and subsequent corrosion caused by the electric arc discharge, thereby improving service life.

In exemplary embodiments, the shielding arrangement comprises a shielding element.

In exemplary embodiments, the shielding element comprises a dielectric material.

In exemplary embodiments, the shielding element comprises quartz.

In exemplary embodiments, the shielding element comprises a hollow body, and the first electrode is positioned within the hollow body of the shielding element.

In exemplary embodiments, the shielding element defines an exterior surface of the elongate body.

In exemplary embodiments, the shielding arrangement comprises first and second shielding elements, and wherein at least part of the flow path, proximal to the gap, is defined by a spacing between the first and second shielding elements.

In exemplary embodiments, the first shielding element comprises a dielectric material.

In exemplary embodiments, the first shielding element comprises quartz.

In exemplary embodiments, the second shielding element comprises a dielectric material.

Advantageously, the dielectric material is able to shield the first and second electrodes from the electric arc discharge.

In exemplary embodiments, the second shielding element comprises quartz.

Advantageously, it has been found that quartz is particularly effective at shielding the first and second electrodes from the electric arc discharge, whilst still enabling an arc discharge to be generated between the electrodes.

In exemplary embodiments, the first shielding element substantially covers the first electrode.

In exemplary embodiments, the second shielding element substantially covers the second electrode.

Advantageously, by covering a substantial part of the first and second electrodes with the first and second shielding elements, the first and second electrodes are better shielded from surface oxidation and subsequent corrosion caused by the electric arc discharge, thereby further improving service life.

In exemplary embodiments, wherein the shielding element defines a length along a longitudinal axis of the elongate body, and wherein the length of the shielding element is greater than a length of the first electrode and/or the second electrode.

In exemplary embodiments, wherein the first shielding element defines a length along a longitudinal axis of the elongate body, and wherein the length of the first shielding element is greater than a length of the second electrode.

In exemplary embodiments, wherein the second shielding element defines a length along a longitudinal axis of the elongate body, and wherein the length of the second shielding element is greater than a length of the second electrode.

Advantageously, by providing shielding elements having a greater length than the arc range of the electric arc discharge (which will not exceed the length of the shortest electrode), the isolation of the electrodes is further improved.

In exemplary embodiments, the first electrode is located within the elongate body.

In exemplary embodiments, the flow path passes around an outer surface of the first electrode.

Advantageously, extending the flow path around a circumference of the first electrode helps to maximise the usable area of the first electrode.

In exemplary embodiments, the first shielding element comprises a hollow body, and the first electrode is positioned within the hollow body of the first shielding element.

In exemplary embodiments, the second shielding element comprises a hollow body, and the first electrode and the first shielding element are positioned within the hollow body of the second shielding element.

In exemplary embodiments, the first and second shielding elements are substantially cylindrical.

In exemplary embodiments, the first and second shielding elements are provided as a pair of concentric cylindrical tubes.

Advantageously, providing the first shielding element and the second shielding element as concentric cylindrical tubes results in a more compact design.

In exemplary embodiments, the second shielding element defines an exterior surface of the elongate body.

Advantageously, by using the second shielding material to form the exterior of the elongate body, the number of parts required for the ozone generator can be effectively reduced, resulting in a more compact design.

In exemplary embodiments, the second electrode is provided at an exterior surface of the elongate body.

Advantageously, such an arrangement allows for easier connection of the second electrode to a power source during use.

In exemplary embodiments, the second electrode extends around at least part of the exterior surface of the elongate body.

Advantageously, extending the second electrode around an exterior surface of the elongate body helps to maximise the usable area of the second electrode.

In exemplary embodiments, the second electrode extends around a substantial portion of the exterior surface of the elongate body.

In exemplary embodiments, the second electrode extends fully around the exterior surface of the elongate body.

In exemplary embodiments, the ozone generator further comprises a second electrode terminal configured for electrically coupling the second electrode to a power source of the ozone generator.

In exemplary embodiments, the second electrode terminal is arranged so as to urge the second electrode against the exterior surface of the elongate body.

In exemplary embodiments, the second electrode conforms around at least a part of the exterior surface of the elongate body.

In exemplary embodiments, the second electrode comprises a metallic foil.

In exemplary embodiments, the second electrode comprises a copper foil.

Advantageously, the use of metallic foil as the second electrode helps the electrode to be easily conformed around the exterior of the elongate body of the ozone generator whilst also providing excellent conductive properties.

In exemplary embodiments, the elongate body comprises an inlet member defining the inlet and a coupling member configured to releasably connect the inlet member to the elongate body.

In exemplary embodiments, the elongate body comprises an outlet member defining the outlet and a coupling member configured to releasably connect the outlet member to the elongate body.

In exemplary embodiments, the coupling member comprises a conductive material.

In exemplary embodiments, the first electrode comprises the coupling member.

Advantageously, by using the coupling member as the first electrode, the number of parts required for the ozone generator can be effectively reduced, resulting in a more compact design.

In exemplary embodiments, the inlet member is configured so as to allow an electrical charge to pass to or from the first electrode during use.

In exemplary embodiments, the outlet member is configured so as to allow an electrical charge to pass to or from the first electrode during use.

In exemplary embodiments, the inlet member and/or outlet member comprise a conductive material.

Advantageously, enabling an electrical charge to be passed to the first electrode via the inlet or outlet allows for easier connection of the first electrode to a power source during use, without requiring additional parts.

In exemplary embodiments, the ozone generator further comprises a first electrode terminal configured for electrically coupling the first electrode to a power source of the ozone generator.

In exemplary embodiments, the first electrode terminal is provided at the inlet member of the ozone generator.

In exemplary embodiments, the first electrode terminal is provided at the outlet member of the ozone generator.

Advantageously, providing the first terminal at the inlet and/or outlet allows for easier connection of the first electrode to a power source during use, without requiring additional parts.

In exemplary embodiments, the inlet member and the coupling member comprise complementary threaded surfaces to releasably secure the inlet member to the coupling member.

In exemplary embodiments, the outlet member and the coupling member comprise complementary threaded surfaces to releasably secure the outlet member to the coupling member.

In exemplary embodiments, the inlet member further comprises a channel arranged so as to allow air travelling along the flow path to pass from the inlet member into the elongate body, and wherein at least a portion of said channel is orientated perpendicular to the longitudinal axis of the inlet member.

Advantageously, orientating the channel perpendicular to the longitudinal axis of the inlet member helps to better convey air traveling along the flow path around the threaded surface of the inlet member and into the elongate body.

In exemplary embodiments, the outlet member further comprises a channel arranged so as to allow ozone gas travelling along the flow path to pass from the elongate body into the outlet member, and wherein at least a portion of said channel is orientated perpendicular to the longitudinal axis of the outlet member.

Advantageously, orientating the channel perpendicular to the longitudinal axis of the outlet member helps to better convey ozone gas traveling along the flow path around the threaded surface of the outlet member and towards the outlet.

In exemplary embodiments, the ozone generator further comprises a sealing element provided between the inlet member and the elongate body, the sealing element being configured so as to substantially prevent the egress of ozone via any gaps present between the elongate body and the inlet and/or outlet members.

In exemplary embodiments, the ozone generator further comprises a sealing element provided between the outlet member and the elongate body, the sealing element being configured so as to substantially prevent the egress of ozone via any gaps present between the elongate body and the inlet and/or outlet members.

Advantageously, the sealing element helps to prevent the egress of ozone via any gaps formed between the elongate body and the inlet / outlet members.

In exemplary embodiments, the sealing element is an O-ring.

In exemplary embodiments, the inlet member comprises a groove, and wherein the sealing element is provided within said groove.

In exemplary embodiments, the outlet member comprises a groove, and wherein the sealing element is provided within said groove.

In exemplary embodiments, the ozone generator is a corona discharge unit type ozone generator.

A second aspect of the teachings provides a hot tub and/or hydrotherapy system comprising the ozone generator according to the first aspect.

### BRIEF DESCRIPTION OF THE DRAWINGS

Embodiments will now be described with reference to the accompanying drawings, in which:
Figure 1 is a cut-away perspective view illustrating an ozone generator according to an embodiment; and
Figure 2 is a perspective view illustrating an exterior of the ozone generator shown in Figure 1.

### DETAILED DESCRIPTION OF EMBODIMENT

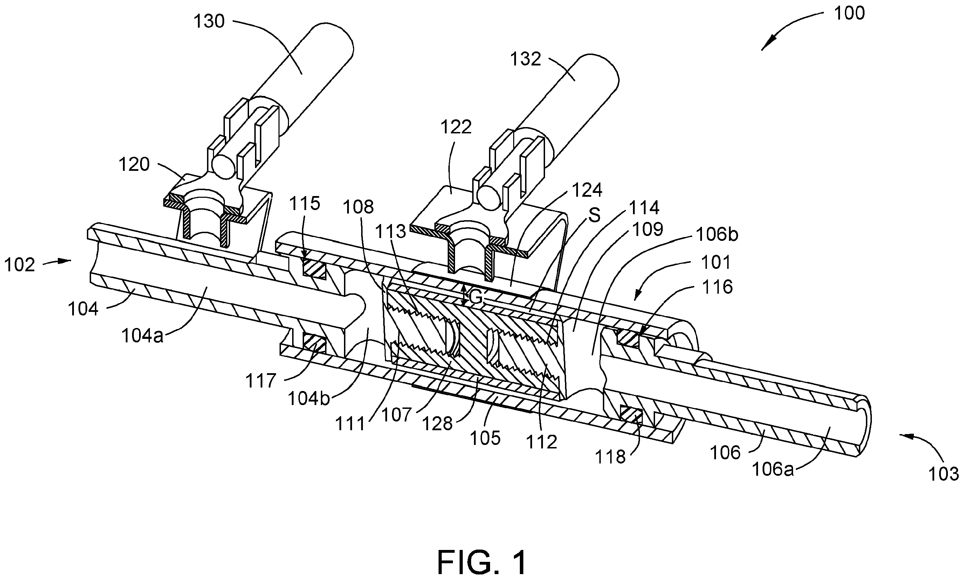

Figure 1 shows an ozone generator 100 for a hot tub or hydrotherapy system (not shown). The ozone generator 100 is a "corona discharge unit" type ozone generator.

The ozone generator 100 comprises an elongate body 101. The elongate body 101 is substantially hollow, having an inlet 102 and an outlet 103 defining a flow path passing through the elongate body 101.

In the illustrated embodiment, the elongate body 101 is an assembly of multiple separate portions. The elongate body 101 is formed from an inlet member 104 defining the inlet 102, an outlet member 106 defining the outlet 103, and a central member 105. The central member 105 is located between the inlet member 104 and the outlet member 106. The central member 105 defines an exterior surface of the elongate body 101. It shall be appreciated that in other embodiments, the inlet member 104 and/or outlet member 106 may be integrally formed with the central member 105 to form the elongate body 101.

The inlet member 104 defines a channel 104a which extends along a longitudinal axis of the inlet member 104. The channel 104a forms an air inlet path between the inlet 102 and the central member 105. The channel 104a extends from the inlet 102 at a first end of the inlet member 104 to a second end of the inlet member 104, distal to the inlet 102. The channel 104a terminates at an end wall 108 and the inlet path includes an exit channel 104b proximal to the end wall 108. The exit channel 104b allows air to flow from the channel 104a into the central member 105. The exit channel 104b is orientated substantially perpendicular to the longitudinal axis of the inlet member 104. The exit channel 104b is open at both ends.

The outlet member 106 defines a channel 106a which extends along a longitudinal axis of the outlet member 106. The channel 106a forms an outlet flow path between the central member 105 and the outlet 103. The outlet flow path includes an entry channel 106b. The entry channel 106b enables ozone generated within the central member 105 to flow from the central member 105 into the channel 106a. The outlet member 106 includes an end wall 109 at a first end thereof. The entry channel 106b is proximal the end wall 109. The entry channel 106b is orientated substantially perpendicular to the longitudinal axis of the outlet member 106. The entry channel is open at both ends. In this way, ozone generated within the central member 105 during use, is able to flow through the outlet member 106 and out of the ozone generator 100 via the outlet 103 to be infused into the water of a hot tub or hydrotherapy system.

The end walls 108, 109 each comprise a threaded surface 111, 112. The threaded surfaces 111, 112 are configured to engage with a respective complementary threaded surface of a coupling member 107 so as to releasably secure the inlet and outlet members 104, 106 to the coupling member 107 of the ozone generator 100. The coupling member 107 is located within the central member 105. In the illustrated embodiment, the coupling member 107 is provided as a substantially cylindrical element. The coupling member 107 includes a pair of threaded surfaces 113 and 114 at each end thereof. This enables the coupling member 107 to releasably engage the threaded surfaces 111, 112 of the inlet and outlet members 104, 106, respectively.

The coupling member 107 is spaced apart from the internal surface of the central member 105 to define a spacing or gap therebetween. Put another way, the coupling member 107 has an external cross-sectional area which is smaller than a cross-sectional area of the inside of the central member 105. This spacing or gap provides a flow path through the central member 105. The spacing is provided around an entirety of the external surface of the coupling member 107. The spacing defines a substantially cylindrical flow path, in the illustrated embodiment. In this way, air is able to pass from the inlet member 104, along the central member 105 and into the outlet member 106.

Whilst the coupling element in the illustrated embodiment is provided as a single part, it shall be appreciated that in other embodiments, the coupling member 107 may be provided as a pair of coupling members, each coupling member having a single threaded surface for releasably engaging with one of the inlet member or outlet member. It shall be appreciated that the inlet and outlet member 104, 106 may be releasably secured directly to the central member 105 using any kind of suitable connection and so, in some embodiments, the coupling member 107 may be omitted.

The inlet and outlet members 104, 106 are each provided with an external groove 115, 116. The external grooves 115, 116 are provided on an external surface of the inlet / outlet member, proximal to the end wall 108, 109. The grooves 115, 116 each house a sealing element 117, 118. In the illustrated embodiment, the sealing element is provided as a rubber O-ring seal. However, in other embodiments, any other suitable form of sealing element may be used. Each sealing element is configured to engage with an interior surface of the central member 105, during use, to prevent the unwanted egress of air and/or ozone gas out of the central member 105.

In the illustrated embodiment, the inlet member 104, outlet member 106 and coupling member 107 are each formed from a conductive material. The conductive material may be a metal. The conductive material may be an ozone resistant material. The conductive materials may be an ozone resistant metallic material, such as steel or copper. However, it shall be appreciated that other suitable materials may be used. For example, the inlet member 104, outlet member 106 and/or coupling member 107 may be formed from an ozone resistant polymer material, such as Silicone or PTFE. Furthermore, in some embodiments, the ozone resistant polymer material may be a conductive polymer material.

The ozone generator 100 includes a pair of electrodes spaced apart to provide a gap (G) therebetween. The gap (G) between the first and second electrodes is provided along the flow path such that, when an electrical charge is applied to the first and second electrodes, an electric arc discharge is generated across the gap (G). This causes any oxygen (O₂) molecules present in the air flowing along the flow path proximal to the gap (G) to break down, upon coming into contact with the electric arc discharge, to form pairs of oxygen atoms (O) which subsequently react with further oxygen molecules (O₂) present in the air flow to form ozone (O₃) ready for introduction into the spa water supply of a hot-tub or hydrotherapy system via the outlet 103 as shall be described in greater detail at a late stage of this application.

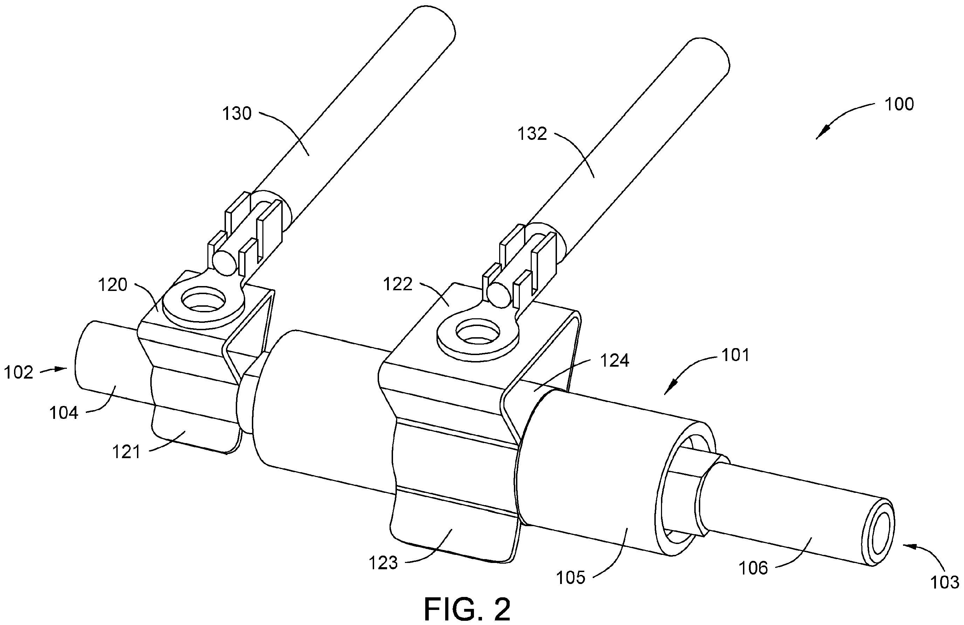

In the illustrated embodiment, the coupling member 107 acts as the first electrode of the ozone generator 100. The coupling member 107 is electrically connected to a power source (not shown) via a first electrode terminal 120. The first electrode terminal 120 is connected to the inlet member 104, as shown in Figure 2. It shall be appreciated that in some embodiments, the first electrode may be directly connected to the first electrode terminal 120. Furthermore, in some embodiments, the first electrode may be provided as a separate electrode element, for example a conductive sheet or plate, rather than being provided by the coupling member.

The first electrode terminal 120 can be clamped onto the inlet member 104 to form a physical and electrical connection between the first electrode terminal 120 and the inlet member 104. The first electrode terminal 120 is provided with a pair of jaws 121 to clamp onto the inlet member 104. During use, an electrical charge can be passed from the power source (not shown), along an electrical cable 130, through the first electrode terminal 120 and along the inlet member 104 to the coupling member 107.

It shall be appreciated that other suitable types of electrical connection may be used to electrically connect the first electrode to the power source. In addition, although the first electrode terminal is provided at the inlet member 104 in the illustrated embodiment, in other embodiments the first electrode terminal may instead be provided at the outlet member 106.

The second electrode 124 is provided on the exterior surface of the elongate body 101. The second electrode is provided at a position that is substantially adjacent to the first electrode (i.e. the coupling member 107). Consequently, the gap (G) between the first and second electrodes intersects the flow path within the central member 105. In the illustrated embodiment, the second electrode 124 is provided as a metallic foil. In some embodiments, the metallic foil may be a copper foil. The second electrode 124 is wrapped around an exterior surface of the elongate body 101 such that the second electrode 124 conforms to the shape of the exterior surface of the elongate body 101.

The second electrode 124 is electrically coupled to a power source (not shown) via a second electrode terminal 122. The second electrode terminal 122 is directly coupled to second electrode 124. The second electrode terminal 122 secures the second electrode 124 in place and forms a physical and electrical connection between the second electrode terminal 122 and the second electrode 124. The second electrode terminal 122 clamps onto the second electrode 124. The second electrode terminal 122 is provided with a pair of jaws 123 arranged to clamp onto the second electrode 124. When the jaws 123 are clamped onto the second electrode 124, they urge the second electrode 124 against the exterior surface of the elongate body 101. In this manner, the second electrode 124 is secured about the elongate body 101. During use, an electrical charge can be passed from the power source (not shown), along an electrical cable 132 and through the second electrode terminal 122 to the second electrode 124.

It shall be appreciated that in other embodiments, other suitable types of electrical connection may be used. For example, the second electrode may be soldered to the electrical cable 132.

The ozone generator 100 includes a shielding arrangement configured to isolate the first and second electrodes 107, 124 from the electric arc discharge. In the illustrated embodiment, the shielding arrangement is provided via first and second shielding elements. However, it shall be appreciated that other suitable arrangements may be used. For example, the shielding arrangement may be provided as a coating applied to the first and second electrodes rather than as separate elements.

The first shielding element 128 is made from a dielectric material. In the illustrated embodiment, the first shielding element is made from quartz. However, it shall be appreciated that other suitable materials may be used.

The first shielding element 128 is provided within the central member 105 of the elongate body 101. The first shielding element 128 has a hollow body. The first shielding element 128 is sized to cover or surround the coupling member 107 (i.e. the first electrode). The first shielding element 128 is sized such that the outer surface of the coupling member 107 is substantially covered by the first shielding element 128. This helps to shield the first electrode from the electric arc discharge generated between the first and second electrodes during use. In the embodiment, the first shielding element 128 is provided as a sleeve (e.g. a substantially cylindrical sleeve) around the coupling member 107. The first shielding element 128 is arranged to abut against an external surface of the coupling member 107 such that there is substantially no spacing there between.

In the illustrated embodiment, the central member 105 forms the second shielding element. The second shielding element is made from a dielectric material. In the illustrated embodiment, the second shielding element is made from quartz. However, it shall be appreciated that other suitable dielectric materials may be used. The coupling member 107 (i.e. the first electrode) and the first shielding element 128 are positioned within the hollow body of the second shielding element. In the illustrated embodiment, the first and second shielding elements are concentric. The first and second shielding elements are provided as a pair of concentric cylindrical tubes. However, it shall be appreciated that in other embodiments, the first and second shielding elements may have any suitable cross-sectional shape and may not necessarily be concentric.

As can be seen in Figure 1, the central member 105 (i.e. the second shielding element) has a length which greatly exceeds that of the second electrode 124. The second electrode is therefore substantially covered by the central member 105. The lengths of the first and second shielding elements along the longitudinal axis of the elongate body 101 are greater than the length of the second electrode. Since the range of the electric arc discharge cannot exceed the length of the shortest electrode, this feature helps to ensure that the first and second electrodes are fully isolated from the electric arc discharge during use.

A spacing (S) is defined between the first and second shielding elements. In the illustrated embodiment, the spacing (S) is provided between the interior surface of the central member 105 (i.e. the second shielding element) and the exterior surface of the first shielding element 128. The spacing (S) forms part of the flow path through the central member 105. The cross-sectional area of the spacing (S), between the first and second shielding elements, is substantially smaller than the cross-sectional area of the respective channels 104a, 106a of the inlet member 104 and outlet member 106.

During operation of the ozone generator, air enters the ozone generator 100 via the inlet 102. Once it has entered the inlet 102, the ambient air passes along the air channel 104a of the inlet member and into the central member 105 via the exit channel 104b. As mentioned previously, the cross-sectional area of the coupling member 107 is smaller than the cross-sectional area of the central member 105 such that air travelling along the flow path, through the central member 105, is able to pass around an outer surface of the coupling member 107. Consequently, as ambient air enters the central member 105 via the inlet member 104, the ambient air passes along the flow path around the outer surface of the coupling member (i.e. the first electrode) through the spacing (S) between the first and second shielding elements.

As the air passes through this space, it will eventually reach a position adjacent to the first and second electrodes wherein the gap (G) between the first and second electrodes intersects the flow path between the first and second shielding elements. When an electrical charge is applied to the first and second electrodes, an electric arc discharge is generated across the gap (G) in the spacing (S) between the first and second shielding elements. This causes any oxygen (O₂) molecules present in the spacing (S) proximal to the gap (G) to form pairs of oxygen atoms (O) upon coming into contact with the electric arc discharge. These oxygen atoms (O) subsequently react with further oxygen molecules (O₂) present in the air flow to form ozone (O₃). The generated ozone (O₃) then exits the spacing (S) and flows out of the central member 105 into the channel 106a of the outlet member 106, via the entry channel 106b. The generated ozone (O₃) can then flow through the outlet 103 ready for infusion into the spa water of a hot tub or hydrotherapy system.

Since the present teachings constrains the electric arc discharge to the spacing (S) between the first and second shielding elements, both the first and second electrodes, as well as other components of the ozone generator 100, are effectively isolated from ionising and oxidising effects of the electric arc discharge. This helps to prevent the corrosion of parts of the ozone generator 100 during use, thereby greatly improving the service life of the ozone generator 100.

Whilst the teachings are envisioned for use as a corona discharge type ozone generator in a hot tub or hydrotherapy system, it shall be appreciated that the aforementioned ozone generator could be used in other applications.

Although the teachings have been described above with reference to one or more preferred embodiments, it will be appreciated that various changes or modifications may be made without departing from the scope as defined in the appended claims.

## Claims

1. An ozone generator for a hot tub, the ozone generator comprising:
an elongate body comprising an inlet and an outlet to define a flow path therealong; and
first and second electrodes spaced apart to define a gap therebetween, said gap being provided along the flow path;
wherein the ozone generator is configured to apply an electrical charge to the first and second electrodes, in use, to generate an electric arc discharge across the gap, and
wherein the ozone generator comprises a shielding arrangement configured to isolate the first and second electrodes from the electric arc discharge generated during use.

2. The ozone generator according to claim 1, wherein the shielding arrangement comprises first and second spaced apart shielding elements, and wherein the electric arc discharge is generated between the first and second shielding elements;
preferably, the first and second shielding elements are interposed between the first and second electrodes.

3. The ozone generator according to claim 2, wherein the first shielding element substantially covers the first electrode and wherein the second shielding element substantially covers the second electrode.

4. The ozone generator according to claim 2 or 3, wherein the first and second shielding elements each define a length along an elongate axis of the elongate body, and wherein the lengths of the first and second shielding elements are greater than a length of the second electrode.

5. The ozone generator according to any of claims 2 to 4, wherein the first and second shielding elements comprise a dielectric material;
preferably, the first and second shielding elements comprise quartz.

6. The ozone generator according to any preceding claim, wherein the first electrode is located within the elongate body and wherein the flow path is arranged around an outer surface of the first electrode.

7. The ozone generator according to claim 6, when dependant on any of claims 2 to 6, wherein the first shielding element comprises a hollow body, and wherein the first electrode is positioned within the hollow body of the first shielding element;
preferably, the second shielding element comprises a hollow body, and wherein the first electrode and the first shielding element are positioned within the hollow body of the second shielding element;
more preferably, the first and second shielding elements are provided as a pair of concentric cylindrical tubes, optionally wherein the second shielding element defines an exterior surface of the elongate body.

8. The ozone generator according to any preceding claim, wherein the second electrode is provided at an exterior surface of the elongate body, optionally wherein the second electrode extends around at least part of the exterior surface of the elongate body;
preferably, the ozone generator further comprises a second electrode terminal configured for electrically coupling the second electrode to a power source of the ozone generator, and wherein the second electrode terminal is arranged so as to urge the second electrode against the exterior surface of the elongate body.

9. The ozone generator according to claim 8, wherein the second electrode conforms around at least a part of the exterior surface of the elongate body;
preferably, the second electrode comprises a metallic foil, optionally a copper foil.

10. The ozone generator according to any preceding claim, wherein the elongate body comprises an inlet member defining the inlet, and/or an outlet member defining the outlet, and a coupling member configured to releasably connect the inlet member and/or the outlet member to the elongate body;
preferably, the coupling member comprises a conductive material;
more preferably, the first electrode comprises the coupling member.

11. The ozone generator according to claim 10, wherein the inlet member and/or outlet member are configured so as to allow an electrical charge to pass to or from the first electrode during use, and preferably wherein the inlet member and/or outlet member comprise a conductive material;
preferably, the ozone generator further comprises a first electrode terminal configured for electrically coupling the first electrode to a power source of the ozone generator, and wherein the first electrode terminal is provided at the inlet member or the outlet member of the ozone generator.

12. The ozone generator according to claim 10 or 11, wherein the inlet member and the coupling member comprise complementary threaded surfaces to releasably secure the inlet member to the coupling member and/or wherein the outlet member and the coupling member comprise complementary threaded surfaces to releasably secure the outlet member to the coupling member.

13. The ozone generator according to claim 12, wherein the inlet member and/or outlet member further comprise a channel arranged so as to allow gas travelling along the flow path to pass from the inlet member into the elongate body and/or from the elongate body into the outlet member, and wherein at least a portion of said channel is orientated perpendicular to the longitudinal axis of the inlet member and/or outlet member.

14. The ozone generator according to any of claims 10 to 13, further comprising a sealing element provided between the inlet member and the elongate body and/or between the outlet member and the elongate body, the sealing element being configured so as to substantially prevent the egress of ozone via any gaps present between the elongate body and the inlet and/or outlet members;
preferably, the inlet member and/or outlet member comprise a groove, and wherein the sealing element is provided within said groove.

15. A hot tub and/or hydrotherapy system comprising the ozone generator according to any preceding claim.
